(19) Europäisches Patentamt
European Patent Office
Office européen des brevets

(11) Publication number: 0 340 755 B1

(12) **EUROPEAN PATENT SPECIFICATION**

(45) Date of publication of patent specification: **01.09.93**

(51) Int. Cl.$^5$: **C07D 211/46**, C07D 207/12, C07D 223/08, C11D 3/395

(21) Application number: **89108022.8**

(22) Date of filing: **03.05.89**

(54) **Monopersulfates of nitrogen containing heterocyclic (poly)percarboxylic acids.**

(30) Priority: **04.05.88 IT 2045688**

(43) Date of publication of application:
**08.11.89 Bulletin 89/45**

(45) Publication of the grant of the patent:
**01.09.93 Bulletin 93/35**

(84) Designated Contracting States:
**AT BE CH DE ES FR GB LI NL SE**

(56) References cited:
**EP-A- 0 056 699**
**EP-A- 0 082 738**
**EP-A- 0 233 476**

**Houben-Weyl, METHODEN DER ORGANISCHEN CHEMIE, vol. III, 1952, Georg Thieme Verlag, Stuttgart**

(73) Proprietor: **AUSIMONT S.p.A.**
**Foro Buonaparte, 31**
**I-20121 Milano(IT)**

(72) Inventor: **Venturello, Carlo, Dr.**
**Via XXIII Marzo 135**
**I-28100 Novara(IT)**
Inventor: **Cavallotti, Claudio**
**Via Lorenteggio 3**
**I-20146 Milano(IT)**
Inventor: **Achilli, Fiorella, Dr.**
**Strada Pianello 6**
**I-29010 Agazzano (PC)(IT)**

(74) Representative: **Weinhold, Peter, Dr. et al**
**Patentanwälte Dr. V. Schmied-Kowarzik**
**Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

EP 0 340 755 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to derivatives of (poly)percarboxylic acids which can be referred to as monopersulfates of nitrogen-containing heterocyclic (poly)percarboxylic acids, to a process for the preparation of said compounds and to their use in bleaching compositions.

In particular, the present invention relates to monopersulfates of nitrogen containing heterocyclic (poly)-percarboxylic acids of general formula (I):

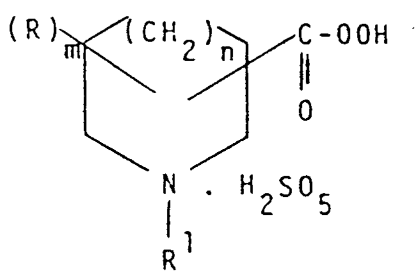

(I)

wherein:
R represents hydrogen,alkyl,cycloalkyl, heterocycloalkyl, aryl, heteroaryl, alkylaryl or arylalkyl, said groups being optionally substituted, a carboxylic (COOH) or a percarboxylic ($CO_3H$) group, or any other substituent non-reactive in the presence of the active oxygen of the peroxycarboxylic group;(if m is 2 or 3 said groups R can be the same or different from each other);
$R^1$ represents an alkyl group having more than 5 carbon atoms;
n is 0,1 or 2;
m is 1, 2 or 3;
and wherein the heterocyclic ring may in its turn be condensed with at least one further (hetero) aromatic or (hetero) cycloalkyl ring.

The derivatives of general formula (I) constitute a new class of products highly interesting from an industrial point of view, particularly due to their high content of active oxygen per weight unit.

In fact, they can find general use, similarly to already known peracids, e.g. in the field of plastics, as polymerization initiators and, in particular, as oxidants for olefin epoxidation and hydroxylation, and in many other oxidative processes in the field of fine chemistry.

Particularly interesting and efficient, however, is their application in the field of bleaching, i.e. in detergents.

In the past years, organic peracids have attracted increasing interest in the industrial field, due to their excellent properties when used as bleaching agents in compositions for medium/low temperature washing, and particularly in view of energy saving considerations.

Consequently, there is considerable research activity aiming at finding new organic peracid compounds having the necessary properties, i.e. high bleaching activity, thermal stability and storage stability or shelf life. The latter requirements are essential for an industrial application and a widespread use of such compounds.

Therefore, many mono- and di-percarboxylic acids, straight-chained or cyclic, are known and used, among others, in the field of detergence.

Already described percarboxylic acids are, e.g.: diperdodecanedioic acid, monoperphthalic acid, diperazelaic acid and substituted diperglutaric and adipic acids,

In conventional preparation processes the oxidation of the substrate is carried out in concentrated $H_2SO_4$ with a solution of hydrogen peroxide.

The above method, when applied to substrates containing salifiable nitrogen atoms of basic character, results in products with a high solubility in the strongly acidic medium.

Said high solubility makes it impossible to apply any of the conventional processes of isolation of the percarboxylic acid derivative which may be formed, such as precipitation and extraction with an organic solvent.

Surprisingly, it has now been found that the nitrogen containing heterocyclic (poly)percarboxylic acid monopersulfates of general formula (I), salified on the nitrogen atom with the persulfuric anion, can be obtained in a stable form by means of a novel process, which is also an object of the present invention.

Therefore, one object of the present invention is to provide novel compounds, i.e. monopersulfates of nitrogen containing heterocyclic (poly)percarboxylic acids having the above formula (I).

Another object of the present invention is to provide a simple and inexpensive process for the preparation of the above percarboxylic acid derivatives having the above formula (I) in a stable form.

A further object of the present invention is the use of the nitrogen containing heterocyclic percarboxylic acid monopersulfates of formula (I) as bleaching agents in detergent formulations, particularly those for low-medium temperature use.

These, and still other objects, which will become evident from the following detailed disclosure, are achieved, according to the present invention, by the monopersulfates of nitrogen containing heterocyclic percarboxylic acids of the above formula (I), and by a process for the preparation thereof which is characterized in that a substrate selected from nitrogen-containing heterocyclic (poly)carboxylic acids or their N-sulfate salts, said substrate corresponding to the desired monopersulfates of formula (I), is reacted with $H_2O_2$ in concentrated $H_2SO_4$ and in that said monopersulfate is then separated from the reaction mixture by means of the addition of an organic solvent selected from tetrahydrofuran and ethyl acetate.

By use of the present process the monopersulfates of formula (I) are generally obtained as stable solids, salified on their nitrogen atom with $H_2SO_5$, due to their insolubilization in the reaction medium by the solvent.

Stated more precisely, the process according to the present invention comprises the percarboxylation reaction of a substrate selected from a nitrogen-containing (poly)carboxylic acid, or its N-sulfate, corresponding to the desired salt of formula (I), in an acidic medium of concentrated $H_2SO_4$ with $H_2O_2$ and the subsequent addition, at the end of the reaction, of a suitable organic solvent which is immiscible with the desired product (by not dissolving it), but which is capable, on the contrary, of completely dissolving the acid reaction medium (concentrated $H_2SO_4$), as well as the excess of $H_2O_2$ and the reaction water. This results in the separation, by insolubilization, of the desired (poly)percarboxylic acid product of formula (I) which usually precipitates in a stable solid form.

The obtained product may then be filtered, washed with the solvent, dried, etc., according to conventional techniques.

As stated above, the substrate used as starting material, is selected from nitrogen-containing heterocyclic (poly)carboxylic acids, or their N-sulfate salts corresponding in structure to the desired (poly)-percarboxylic acid monopersulfate of formula (I); these substrates are known, and/or can be prepared according to known processes.

Referring to the above formula (I), R preferably is a linear or branched alkyl, (hetero)-aryl, (hetero)-cycloalkyl, alkyl-aryl or aryl-alkyl group, containing an overall number of up to 10, particularly up to 6, carbon atoms, and, in the heterocyclic rings, the presence of N atoms and/or 0 atoms (e.g. 1 or 2 such hetero atoms) is preferred.

Said groups may in turn be substituted with one or more (preferably 1 to 3) atoms or groups, the same or different from each other and inert under the reaction conditions under which the preparation takes place, such as, e.g., F, Cl, $NO_2$, $C_{1-5}$ alkoxy.

Alternatively, R represents any other substituent which does not react with the active oxygen of the percarboxylic group, e.g., a carboxylic group, a percarboxylic group, F, Cl, $NO_2$, $(C_{1-5})$-alkoxy,

$R_1$ represents a linear or branched alkyl group containing more than 5 carbon atoms, preferably having 8 to 20, and particularly 8 to 16, carbon atoms.

The above heterocyclic ring may optionally be condensed with at least one other (hetero) aromatic or (hetero) cycloalkyl ring and thus be in the form of e.g. a quinolinic, isoquinolinic, pyrido-indolic group,
n is preferably equal to 1, whereas a preferred meaning for R is hydrogen.

Specific examples of the generic groups mentioned above are:

| | |
|---|---|
| alkyl: | methyl, ethyl, n- and i-propyl, n-, i-. sec.- and tert.butyl, pentyl, hexyl, heptyl, octyl, nonyl, decyl, undecyl, dodecyl, tridecyl, tetradecyl, pentadecyl, hexadecyl and octadecyl; |
| aryl: | phenyl, naphthyl, biphenylyl and anthracenyl; |
| heteroaryl: | pyrrolyl, imidazolyl, furanyl, pyridinyl, pyranyl, pyrazinyl, indolyl, quinolinyl and isoquinolinyl; |
| cycloalkyl: | cyclopentyl, cyclohexyl, cycloheptyl and cyclooctyl; |
| heterocycloalkyl: | tetrahydrofuranyl, pyrrolidinyl, imidazolidinyl, piperidyl, tetrahydropyranyl and morpholinyl; |
| alkylaryl: | tolyl, xylyl, ethylphenyl and methylnaphthyl; |
| arylalkyl: | benzyl, phenethyl and naphthylmethyl; |
| alkoxy: | methoxy, ethoxy, propoxy, butoxy and pentoxy. |

Examples of suitable substrates are, e.g., N-octyl-4-piperidine carboxylic acid, N-decyl-4-piperidinecarboxylic acid and N-hexadecyl-4-piperidinecarboxylic acid.

For R = COOH, preferably in a non-ortho-position with respect to the nitrogen atom, the percarboxylation of R can be carried out, too, thus resulting in a product of formula (I) with two or more (at the most four) percarboxylic groups.

According to a preferred mode of operation, the percarboxylation reaction of the nitrogen containing heterocyclic (poly)carboxylic acids or the corresponding sulfates used as starting material, is carried out by gradually adding $H_2O_2$ having a concentration within the range of from 70% to 90% by weight to a solution of the substrate in concentrated $H_2SO_4$ (96-98%), maintaining the reaction temperature throughout the reaction at a temperature lower than 20 °C.
Alternatively, it has been found that it is possible to salify (as $H_2SO_4$ salt) the substrate, by working in the absence of $H_2O_2$ under otherwise the same conditions as described above, and to separate the obtained salt first, said salt being thereafter peroxidized.

The amount of $H_2SO_4$, based on a concentration of 100%, is preferably at least 5 moles per mole of substrate, and most preferred ranges are from 6 to 15 moles.

Hydrogen peroxide is preferably used in an amount which is in excess with respect to the substrate and preferably equals at least 5 moles per mole of substrate.

The reaction time depends on the nature of the subtrate, the operating temperature, and the final total $H_2SO_4/H_2O$molar ratio present at the end of the reaction. Said ratio is preferably chosen so as to be of from 1.3 to 4 by adjusting the various parameters concerned.

Reaction times of from 30 minutes to 1 hour have proven to be the usual ones.

The employed amount of tetrahydrofuran and/or ethyl acetate solvent is preferably not lower than 4 litres/mole of substrate, e.g., 7 litres/mole; furthermore, the solvent is preferably added at a temperature not higher than 10 °C.

The nitrogen-containing heterocyclic percarboxylic acid monopersulfates of formula (I) usually are solid at room temperature. They can be used to particular advantage in formulations of detergent compositions, e.g., granular formulations,and as bleaching agents in solution within a wide temperature range, due to their high storage stability and thermal stability.

The detergent compositions can be formulated according to the usual pertinent techniques, using further components and/or additives.

The present invention will now be described in more detail by the following examples.

The products prepared in the examples were characterized by elemental analysis, by determining their content of active oxygen (by iodometric titration), and by using the Fourier Transform Infrared Spectroscopy (FT-IR).

EXAMPLE 1

5.1 g (0.019 mole) of N-decyl-isonipecotic acid were completely dissolved, at +35 °C, in 11.6 g of $H_2SO_4$ (96%, 0.114 mole).
3.8 g of $H_2O_2$ (85%, 0.095 mole) were then added to the solution so that the temperature was maintained at or below +15 °C.
Stirring was then continued for 45 minutes at +15 °C.

The reaction mixture was then poured into 140 ml of ethyl acetate maintained under stirring at -10 °C. After 30 minutes the separated crystalline product was filtered under vacuum over a porous septum and was directly washed on the filter with consecutive portions of ethyl acetate (2 x 30 ml) and ethyl ether (2 x 30 ml). The product was then kept in a $CaCl_2$-drier under vacuum and at room temperature for 1 hour.

Obtained were 4.8 g of crystalline N-decyl-piperidine-4-percarboxylic acid having an active oxygen content of 7.8% (97.4% of the theoretical value). Yield: 62%.

Elemental Analysis:

Calculated for $C_{16}H_{33}NSO_8$: C 48.10%; H 8.32%; N 3.50%; 0 (active) 8.01; $H_2SO_5$ 28.55%.
Found: C 48.06%; H 8.41%; N 3.49%; 0 (active) 7.80%; $H_2SO_5$ 28.5%.
Melting point: 78 °C (with decomposition).

EXAMPLE 2

4 g (0.0113 mole) of N-hexadecyl-4-piperidine-carboxylic acid were added, slowly and under stirring, to 2.5 g of sulphuric acid in a 25 ml beaker, care being taken to maintain the temperature at or below 40 °C by use of a cooling bath. Stirring was then continued at 35 to 40 °C for 2 hours.

The reaction mixture was then poured into 150 ml of ethyl acetate maintained under stirring at 10 °C. Stirring was then continued for 30 minutes.

The separated N-hexadecyl-4-piperidine-carboxylic acid sulfate was filtered over a porous septum, washed first with ethyl acetate (2 x 30 ml) and then with $Et_20$ (2 x 30 ml), and thereafter dried under vacuum at room temperature over $CaCl_2$.

Obtained were 4.1 g of product which was used for preparing the corresponding peracid monopersulfate.

2 g of $H_2O_2$ (85%, 0.05 mole) were added, under stirring, to 12 g of $H_2SO_4$ (96%, 0.1175 mole) by maintaining the temperature at or below +5 °C. 4.1 g of N-hexadecyl-isonipecotic acid sulfate (0.0091 mole) were added so as to maintain the temperature at or below +15 °C. Stirring was then continued at +15 °C for 30 minutes. The reaction mixture was poured into 100 ml of ethyl acetate maintained under stirring at -10 °C. Thereafter the procedure of example 1 was followed.

3.2 g of crystalline, practically pure N-hexadecylpiperidine-4-percarboxylic acid monopersulfate were obtained. Yield: 73%.

Elemental Analysis:

Calculated for $C_{22}H_{45}NSO_8$: C 54.63%; H 9.37%; N 2.89%; O (active) 6.61%; $H_2SO_5$ 23.58%.
Found: C 53.9%; H 9.35%; N 2.89%; O (active) 6.6%; $H_2SO_5$ 23.41%.
Melting point: 84 °C (with decomposition).

EXAMPLE 3 (Application Example)

Bleaching tests were carried out with the novel salt listed in the annexed Tables 1 and 2, at alkaline pH (Table 1) and acid pH (Table 2). The following substance served as comparison:
H 48 (Mg salt of monoperphthalic acid), a commercial peracid known in the detergent art, and manufactured by INTEROX Chemical Ltd., London, U.K. (Tables 1 and 2).

All tests were carried out at constant temperature (60 °C), with an initial concentration of total active oxygen in the bleaching solution (identical for all products) of equal to 200 mg/l.

Process

For each test, 500 ml of deionized water, contained in a 1000 ml flask equipped with a condenser, was heated to a temperature of 60 °C and adjusted to a pH of 9.5 (by adding a few drops of an NaOH solution) (Table 1) and of 3 - 4 (by adding a few drops of diluted $H_2SO_4$)(Table 2). Then the bleaching product was added with stirring, in amounts shown in the following Tables, and immediately thereafter two cotton specimens of 10 cm x 10 cm,stained with standard stains of red wine at EMPA INSTITUTE of St.Gallen (Switzerland), and marked with the "EMPA 114" mark, were added.

The system subsequently was kept under stirring for 60 minutes and, at the end of this time, the specimens, rinsed under running water, were dried and ironed, and were then subjected to an evaluation of the bleaching effect by means of a measurement of the whiteness degree by reflectometry. The results are reported in the following Tables 1 and 2, wherein the data are expressed as % Bleaching, defined as:

$$\%\ \text{Bleaching} = \frac{A - B}{C - B} \times 100\%$$

wherein:

A = degree of whiteness (%) of the specimen bleached after the test;
B = degree of whiteness (%) of the specimen before the test;
C = degree of whiteness (%) of the completely bleached specimen.

The degree of whiteness was measured by means of an Elrepho Zeiss reflectometer, assuming MgO = 100% of whiteness, and using a filter N.6 ($\lambda$ = 464 nm).

The data listed in Table 1, concerning tests carried out at alkaline pH, show that the peracid salts of the present invention have a bleaching power which may be compared to the bleaching power of H 48.

Likewise, the results, expressed as % Bleaching, listed in Table 2, show that the tested products have a bleaching power in acid solution which is particularly high and higher than that of H 48.

This is particularly surprising in consideration of the fact that the peroxidic compounds generally show a very modest and sometimes negligible bleaching activity at acid pH.

TABLE 1

| Test carried out at alkaline pH (9.5) | | | |
|---|---|---|---|
| Compound | Amounts used in the tests (grams) | Initial concentration of total active oxygen (mg/l) | % Bleaching |
| - Example 1 (titer = 5.50% of active oxygen) | 1.28 | 200 | 74.18 |
| - H 48 (titer = 5.5% of active oxygen) | 1.86 | 200 | 81.0 |

TABLE 2

| Test carried out at acid pH (3-4) | | | |
|---|---|---|---|
| Compound | Amounts used in the tests (grams) | Initial concentration of total active oxygen (mg/l) | % Bleaching |
| - Example 1 (titer = 7.80% of active oxygen) | 1.28 | 200 | 78.4 |
| - H 48 (titer = 5.5% of active oxygen) | 1.86 | 200 | 60.0 |

## Claims

**Claims for the following Contacting States : AT, BE, CH, DE, FR, GB, LI, NL, SE**

**1.** Monopersulfates of nitrogen containing heterocyclic (poly)percarboxylic acids of general formula:

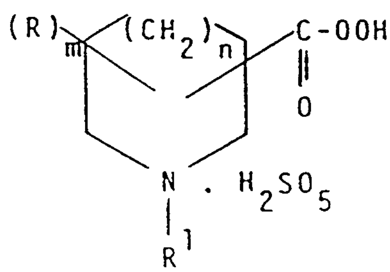

(I)

wherein:

R represents hydrogen or linear or branched alkyl, (hetero)aryl, (hetero) cycloalkyl, alkylaryl or arylalkyl containing an overall number of up to 10 carbon atoms, said groups being optionally substituted by at least one substituent selected from F, Cl, $NO_2$ or ($C_1$-$C_5$)-alkoxy; a carboxylic or a percarboxylic group; a F atom, a Cl atom and a ($C_1$-$C_5$)-alkoxy group, and wherein the heteroatoms are selected from N and O;

$R^1$ represents an alkyl group having more than 5 carbon atoms;

n is an integer selected from 0, 1 and 2;

m is an integer selected from 1, 2 and 3;

and wherein the heterocyclic ring itself may optionally be condensed with at least one further (hetero)-aromatic or (hetero)cycloalkyl ring, the heteroatoms being selected from N and O.

2. Monopersulfates according to claim 1, wherein $R^1$ represents an alkyl group containing from 8 to 20 carbon atoms.

3. Monopersulfates according to any one of claims 1 and 2, wherein n is 1 and R is hydrogen.

4. Monopersulfates according to any one of claims 1 to 3, wherein the heterocyclic ring is condensed with at least one other (hetero)aromatic or (hetero)cycloalkyl pyridinic ring.

5. compounds according to claim 1, i.e. N-decyl-piperidine-4-percarboxylic acid monopersulfate and N-hexadecylpiperidine-4-percarboxylic acid monopersulfate.

6. Process for preparing the monopersulfates according to claim 1, characterized in that a substrate selected from a nitrogen containing heterocyclic (poly)carboxylic acid and its N-sulfate salt, corresponding to the desired percarboxylic acid of formula (I), is reacted with $H_2O_2$ in a concentrated $H_2SO_4$ medium and that the resulting monopersulfate of general formula (I) is separated from the reaction mixture by addition of tetrahydrofuran and/or ethyl acetate.

7. Process according to claim 6, characterized in that a nitrogen containing heterocyclic (poly)-percarboxylic acid corresponding to the desired compound of formula (I) is converted into the corresponding $H_2SO_4$ salt which is then reacted with $H_2O_2$ in concentrated $H_2SO_4$.

8. Process according to any one of claims 6 and 7, characterized in that the heterocyclic nitrogen containing (poly)carboxylic acid or its N-sulfate'salt is gradually reacted with $H_2O_2$ having a concentration of from 70% to 90% by weight in concentrated $H_2SO_4$ at a temperature lower than 20 °C.

9. Process according to any one of claims 6 to 8, characterized in that the amount of $H_2SO_4$ is at least equal to 5 moles per mole of substrate.

10. Process according to claim 9, characterized in that the amount of $H_2SO_4$ ranges from 6 to 15 moles per mole of substrate.

11. Process according to any one of the claims 6 to 10, characterized in that an amount of hydrogen peroxide is used which is at least equal to 5 moles per mole of substrate.

12. Process according to any one of claims 6 to 11, characterized in that the final molar ratio of $H_2SO_4$ to the total $H_2O$ present at the end of the reaction ranges from 1.3 to 4.

13. Process according to any one of claims 6 to 12, characterized in that the amount of tetrahydrofuran and/or ethyl acetate used is not lower than 4 litres per mole of substrate.

14. Process according to any one of claims 6 to 13, characterized in that the tetrahydrofuran and/or ethyl acetate solvent is added at a temperature not higher than 10 °C.

15. Use of monopersulfates according to any one of claims 1 to 5 as bleaching agents in detergent formulations.

**Claims for the following Contracting State : ES**

1. Process for preparing monopersulfates of nitrogen containing heterocyclic (poly)percarboxylic acids of general formula:

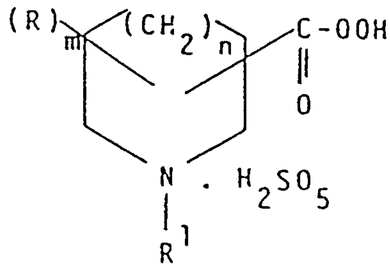

(I)

wherein:

R represents hydrogen or linear or branched alkyl, (hetero)aryl, (hetero) cycloalkyl, alkylaryl or arylalkyl containing an overall number of up to 10 carbon atoms, said groups being optionally Substituted by at least one substituent selected from F, Cl, $NO_2$ or ($C_1$-$C_5$)-alkoxy; a carboxylic or a percarboxylic group; a F atom, a Cl atom and a ($C_1$-$C_5$)-alkoxy group, and wherein the heteroatoms are selected from N and O;

$R^1$ represents an alkyl group having more than 5 carbon atoms;

n is an integer selected from 0, 1 and 2;

m is an integer selected from 1, 2 and 3;

and wherein the heterocyclic ring itself may optionally be condensed with at least one further (hetero)-aromatic or (hetero)cycloalkyl ring, the heteroatoms being selected from N and O,

characterized in that a substrate selected from a nitrogen containing heterocyclic (poly)carboxylic acid and its N-sulfate salt, corresponding to the desired percarboxylic acid of formula (I), is reacted with $H_2O_2$ in a concentrated $H_2SO_4$ medium and that the resulting monopersulfate of general formula (I) is separated from the reaction mixture by addition of tetrahydrofuran and/or ethyl acetate.

**2.** Process according to claim 1, wherein $R^1$ represents an alkyl group containing from 8 to 20 carbon atoms.

**3.** Process according to any of the claims 1 and 2, wherein n is 1 and R is hydrogen.

**4.** Process according to any one of claims 1 to 3, wherein the heterocyclic ring is condensed with at least one other (hetero)aromatic or (hetero)cycloalkyl pyridinic ring.

**5.** Process according to claim 1, wherein the monopersulfates are N-decyl-piperidine-4-percarboxylic acid monopersulfate and N-hexadecyl-piperidine-4-percarboxylic acid monopersulfate.

**6.** Process according to any one of claims 1 to 5, characterized in that a nitrogen containing heterocyclic (poly)percarboxylic acid corresponding to the desired compound of formula (I) is converted into the corresponding $H_2SO_4$ salt which is then reacted with $H_2O_2$ in concentrated $H_2SO_4$.

**7.** Process according to any one of claims 1 to 6, characterized in that the heterocyclic nitrogen containing (poly)carboxylic acid or its N-sulfate salt is gradually reacted with $H_2O_2$ having a concentration of from 70% to 90% by weight in concentrated $H_2SO_4$ at a temperature lower than 20 °C.

**8.** Process according to any one of claims 1 to 7, characterized in that the amount of $H_2SO_4$ is at least equal to 5 moles per mole of substrate.

**9.** Process according to claim 8, characterized in that the amount of $H_2SO_4$ ranges from 6 to 15 moles per mole of substrate.

**10.** Process according to any one of the claims 1 to 9, characterized in that an amount of hydrogen peroxide is used which is at least equal to 5 moles per mole of substrate.

**11.** Process according to any one of claims 1 to 10, characterized in that the final molar ratio of $H_2SO_4$ to the total $H_2O$ present at the end of the reaction ranges from 1.3 to 4.

12. Process according to any one of claims 1 to 11, characterized in that the amount of tetrahydrofuran and/or ethyl acetate used is not lower than 4 litres per mole of substrate.

13. Process according to any one of claims 1 to 12, characterized in that the tetrahydrofuran and/or ethyl acetate solvent is added at a temperature not higher than 10 ° C.

14. Use of monopersulfates obtained according to any one of claims 1 to 13 as bleaching agents in detergent formulations.

**Patentansprüche**
**Patentansprüche für folgende Vertragsstaaten : AT, BE, CH, DE, FR, GB, LI, NL, SE**

1. Monopersulfate stickstoffhaltiger heterocyclischer (Poly)percarbonsäuren der allgemeinen Formel (I):

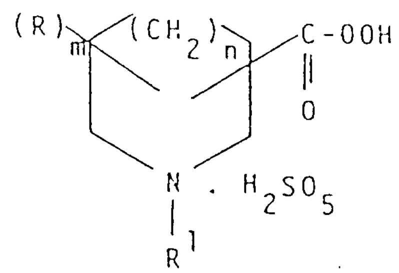

I

worin
R bedeutet: Wasserstoff oder lineares oder verzweigtkettiges Alkyl, (Hetero)aryl, (Hetero)cycloalkyl, Alkylaryl oder Arylalkyl mit einer Gesamtzahl von bis zu 10 Kohlenstoffatomen, wahlweise substituiert mit wenigstens einem Substituenten, ausgewählt aus F, Cl, $NO_2$ oder ($C_1$-$C_5$)-Alkoxy; eine Carboxyl- oder eine Percarboxylgruppe; ein F-Atom, ein Cl-Atom und eine ($C_1$-$C_5$)-Alkoxygruppe, und worin die Heteroatome ausgewählt sind aus N und O;
$R^1$ eine Alkylgruppe mit mehr als 5 Kohlenstoffatomen bedeutet;
n eine ganze Zahl ausgewahlt aus 0, 1 und 2 ist;
m eine ganze Zahl ausgewählt aus 1, 2 und 3 ist;
und worin der heterocyclische Ring seinerseits wahlweise mit wenigstens einem weiteren (hetero)-aromatischen Ring oder (Hetero)cycloalkyl-Ring kondensiert sein kann, wobei die Heteroatome aus N und O ausgewählt sind.

2. Monopersulfate nach Anspruch 1, worin $R^1$ eine Alkylgruppe mit 8 bis 20 Kohlenstoffatomen bedeutet.

3. Monopersulfate nach irgendeinem der Ansprüche 1 und 2, worin n gleich 1 ist und R Wasserstoff bedeutet.

4. Monopersulfate nach irgendeinem der Ansprüche 1 bis 3, worin der heterocyclische Ring mit wenigstens einem weiteren (hetero)aromatischen oder (Hetero)cycloalkyl-pyridinring kondensiert ist.

5. Verbindungen nach Anspruch 1, z.B. N-Decyl-piperidin-4-percarbonsäuremonopersulfat und N-Hexadecyl-piperidin-4-percarbonsäuremonopersulfat.

6. Verfahren zur Herstellung der Monopersulfate nach Anspruch 1, dadurch gekennzeichnet, daß ein Substrat, ausgewählt aus einer stickstoffhaltigen heterocyclischen (Poly)carbonsäure und ihrem N-Sulfatsalz, entsprechend der gewünschten Percarbonsäure der Formel (I), mit $H_2O_2$ in konzentriertem $H_2SO_4$-Medium umgesetzt wird und daß das resultierende Monopersulfat der allgemeinen Formel (I) aus der Reaktionsmischung durch Zugabe von Tetrahydrofuran und/oder Ethylacetat abgetrennt wird.

7. Verfahren nach Anspruch 6, dadurch gekennzeichnet, daß eine stickstoffhaltige heterocyclische (Poly)-percarbonsäure, die der gewünschten Percarbonsäure der Formel (I) entspricht, in das entsprechende $H_2SO_4$-Salz überführt wird, das anschließend mit $H_2O_2$ in konzentrierter $H_2SO_4$ umgesetzt wird.

8. Verfahren nach irgendeinem der Ansprüche 6 und 7, dadurch gekennzeichnet, daß die stickstoffhaltige heterocyclische (Poly)carbonsäure oder ihr N-Sulfatsalz nach und nach bei einer Temperatur von unter 20 °C mit $H_2O_2$ mit einer Konzentration von 70 bis 90 Gew.-% in konzentrierter $H_2SO_4$ umgesetzt wird.

9. Verfahren nach irgendeinem der Ansprüche 6 bis 8, dadurch gekennzeichnet, daß die Menge an $H_2SO_4$ wenigstens 5 Mol pro Mol Substrat beträgt.

10. Verfahren nach Anspruch 9, dadurch gekennzeichnet, daß die Menge an $H_2SO_4$ zwischen 6 und 15 Mol pro Mol Substrat liegt.

11. Verfahren nach irgendeinem der Ansprüche 6 bis 10, dadurch gekennzeichnet, daß die Menge an eingesetztem Wasserstoffperoxid wenigstens 5 Mol pro Mol Substrat beträgt.

12. Verfahren nach irgendeinem der Ansprüche 6 bis 11, dadurch gekennzeichnet, daß das End-Molverhältnis von $H_2SO_4$ zu gesamtem $H_2O_2$, das am Ende der Reaktion vorliegt, im Bereich von 1,3 bis 4 liegt.

13. Verfahren nach irgendeinem der Ansprüche 6 bis 12, dadurch gekennzeichnet, daß die Menge an eingesetztem Tetrahydrofuran und/oder Ethylacetat nicht weniger als 4 Liter pro Mol Substrat beträgt.

14. Verfahren nach irgendeinem der Ansprüche 6 bis 13, dadurch gekennzeichnet, daß das Lösungsmittel Tetrahydrofuran und/oder Ethylacetat bei einer Temperatur zugegeben wird, die nicht höher als 10 °C liegt.

15. Verwendung der Monopersulfate nach irgendeinem der Ansprüche 1 bis 5 als Bleichmittel in Detergensformulierungen.

**Patentansprüche für folgenden Vertragsstaat : ES**

1. Verfahren zur Herstellung von Monopersulfaten stickstoffhaltiger heterocyclischer (Poly)-percarbonsäuren der allgemeinen Formel (I):

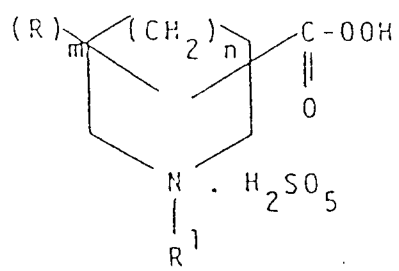

I

worin
R bedeutet: Wasserstoff oder lineares oder verzweigtkettiges Alkyl, (Hetero)aryl, (Hetero)cycloalkyl, Alkylaryl oder Arylalkyl mit einer Gesamtzahl von bis zu 10 Kohlenstoffatomen, wahlweise substituiert mit wenigstens einem Substituenten, ausgewählt aus F, Cl, $NO_2$ oder ($C_1$-$C_5$)-Alkoxy; eine Carboxyl- oder eine Percarboxylgruppe; ein F-Atom, ein Cl-Atom und eine ($C_1$-$C_5$)-Alkoxygruppe, und worin die Heteroatome ausgewahählt sind aus N und O;
$R^1$ eine Alkylgruppe mit mehr als 5 Kohlenstoffatomen bedeutet;
n eine ganze Zahl ausgewählt aus 0, 1 und 2 ist;
m eine ganze Zahl ausgewählt aus 1, 2 und 3 ist;
und worin der heterocyclische Ring seinerseits wahlweise mit wenigstens einem weiteren (hetero)-aromatischen Ring oder (Hetero)cycloalkyl-Ring kondensiert sein kann, wobei die Heteroatome aus N und O ausgewählt sind,
dadurch gekennzeichnet, daß ein Substrat, ausgewählt aus einer stickstoffhaltigen heterocyclischen (Poly)carbonsäure und ihrem N-Sulfatsalz, entsprechend der gewünschten Percarbonsäure der Formel

(I), mit $H_2O_2$ in konzentriertem $H_2SO_4$-Medium umgesetzt wird und daß das resultierende Monopersulfat der allgemeinen Formel (I) aus der Reaktionsmischung durch Zugabe von Tetrahydrofuran und/oder Ethylacetat abgetrennt wird.

**2.** Verfahren nach Anspruch 1, worin $R^1$ eine Alkylgruppe mit 8 bis 20 Kohlenstoffatomen bedeutet.

**3.** Verfahren nach irgendeinem der Anspruche 1 und 2, worin n gleich 1 ist und R Wasserstoff bedeutet.

**4.** Verfahren nach irgendeinem der Ansprüche 1 bis 3, worin der heterocyclische Ring mit wenigstens einem weiteren (hetero)aromatischen oder (Hetero)cycloalkyl-pyridinring kondensiert ist.

**5.** Verfahren nach Anspruch 1, worin die Monopersulfate N-Decyl-piperidin-4-percarbonsäuremonopersulfat und N-Hexadecyl-piperidin-4-percarbonsäuremonopersultat sind.

**6.** Verfahren nach irgendeinem der Ansprüche 1 bis 5, dadurch gekennzeichnet, daß eine stickstoffhaltige heterocyclische (Poly)percarbonsäure, die der gewünschten Percarbonsäure der Formel (I) entspricht, in das entsprechende $H_2SO_4$-Salz überführt wird, das anschließend mit $H_2O_2$ in konzentrierter $H_2SO_4$ umgesetzt wird.

**7.** Verfahren nach irgendeinem der Ansprtiche 1 bis 6, dadurch gekennzeichnet, daß die stickstoffhaltige heterocyclische (Poly)carbonsäure oder ihr N-Sulfatsalz nach und nach bei einer Temperatur von unter 20 °C mit $H_2O_2$ in einer Konzentration von 70 bis 90 Gew.-% in konzentrierter $H_2SO_4$ umgesetzt wird.

**8.** Verfahren nach irgendeinem der Ansprüche 1 bis 7, dadurch gekennzeichnet, daß die Menge an $H_2SO_4$ wenigstens 5 Mol pro Mol Substrat beträgt.

**9.** Verfahren nach Anspruch 8, dadurch gekennzeichnet, daß die Menge an $H_2SO_4$ zwischen 6 und 15 Mol pro Mol Substrat liegt.

**10.** Verfahren nach irgendeinem der Ansprüche 1 bis 9, dadurch gekennzeichnet, daß die Menge an eingesetztem Wasserstoffperoxid wenigstens 5 Mol pro Mol Substrat beträgt.

**11.** Verfahren nach irgendeinem der Ansprüche 1 bis 10, dadurch gekennzeichnet, daß das End-Molverhältnis von $H_2SO_4$ zu gesamtem $H_2O_2$, das am Ende der Reaktion vorliegt, im Bereich von 1,3 bis 4 liegt.

**12.** Verfahren nach irgendeinem der Arsprüche 1 bis 11, dadurch gekennzeichnet, daß die Menge an eingesetztem Tetrahydrofuran und/oder Ethylacetat nicht weniger als 4 Liter pro Mol Substrat beträgt.

**13.** Verfahren nach irgendeinem der Ansprüche 1 bis 12, dadurch gekennzeichnet, daß das Lösungsmittel Tetrahydrofuran und/oder Ethylacetat bei einer Temperatur zugegeben wird, die nicht höher als 10 °C liegt.

**14.** Verwendung der nach irgendeinem der Ansprüche 1 bis 13 erhaltenen Monopersulfate als Bleichmittel in Detergensformulierungen.

**Revendications**

**Revendications pour les Etats contractants suivants : AT, BE, CH, DE, FR, GB, LI, NL, SE**

**1.** Monopersulfates d'acides (poly)-percarboxyliques hétérocycliques azotés de formule générale (I):

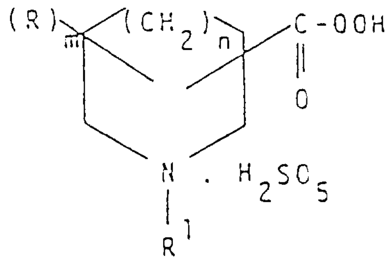

dans laquelle:

R représente un atome d'hydrogène, ou un groupe alkyle linéaire ou ramifié, (hétéro)aryle, (hétéro)cycloalkyle, alkylaryle ou arylalkyle contenant jusqu'à 10 atomes de carbone, ces groupes étant éventuellement substitués par au moins un substituant choisi parmi F, Cl, $NO_2$ ou un groupe alcoxy en $C_1$-$C_5$; un groupe carboxylique ou un groupe percarboxylique; un atome de F, un atome de Cl et un groupe alcoxy en $C_1$-$C_5$, et dans laquelle les hétéroatomes sont sélectionnés parmi N et O;

$R^1$ représente un groupe alkyle ayant plus de 5 atomes de carbone;

n est un nombre entier choisi parmi 0, 1 et 2;

m est un nombre entier choisi parmi 1, 2 et 3; et dans laquelle le cycle hétérocyclique peut lui-même être éventuellement condensé avec au moins un autre cycle (hétéro)aromatique ou (hétéro)cycloalkyle, les hétéroatomes étant sélectionnés parmi N et O.

**2.** Monopersulfates selon la revendication 1, dans lesquels $R^1$ représente un groupe alkyle contenant de 8 à 20 atomes de carbone.

**3.** Monopersulfates selon l'une quelconque des revendications 1 et 2, dans lesquelles n est égal à 1 et R est un atome d'hydrogène.

**4.** Monopersulfates selon l'une quelconque des revendications 1 à 3 dans lesquelles le cycle hétérocyclique est condensé avec au moins un autre cycle (hétéro)aromatique ou (hétéro)cycloalkyle pyridinique.

**5.** Composés selon la revendication 1, c'est-à-dire monopersulfate d'acide N-décyl-pipéridine-4-percarboxylique et monopersulfate d'acide N-hexadécyl-pipéridine-4-percarboxylique.

**6.** Procédé pour la préparation de monopersulfates selon la revendication 1, caractérisé en ce que le substrat choisi parmi les acides (poly)carboxyliques hétérocycliques azotés et leur sel de N-sulfate, correspondant aux acides percarboxyliques désirés de formule (I), réagit avec $H_2O_2$ dans un milieu $H_2SO_4$ concentré et que le monopersulfate de formule générale (I) résultant est séparé du milieu réactionnel par addition de tétrahydrofuranne et/ou acétate d'éthyle.

**7.** Procédé selon la revendication 6, caractérisé en ce que l'acide (poly)percarboxylique hétérocyclique azoté correspondant au composé désiré de formule (I) est converti en son sel d'$H_2SO_4$ correspondant qui réagit alors avec $H_2O_2$ dans $H_2SO_4$ concentré.

**8.** Procédé selon l'une quelconque des revendications 6 et 7, caractérisé en ce que l'acide (poly)-carboxylique hétérocyclique azoté ou son sel de N-sulfate réagit de façon graduelle avec $H_2O_2$ ayant une concentration de 70 à 90% en poids dans $H_2SO_4$ concentré à une température inférieure à 20 °C.

**9.** Procédé selon l'une quelconque des revendications 6 à 8, caractérisé en ce que la quantité de $H_2SO_4$ est au moins égale à 5 moles par mole de substrat.

**10.** Procédé selon la revendication 9, caractérisé en ce que la quantité de $H_2SO_4$ est comprise dans un intervalle allant de 6 à 15 moles par mole de substrat.

**11.** Procédé selon l'une quelconque des revendications 6 à 10, caractérisé en ce que la quantité de peroxyde d'hydrogène utilisée est au moins égale à 5 moles par mode de substrat.

**12.** Procédé selon l'une quelconque des revendications 6 à 11, caractérisé en ce que le rapport molaire final de $H_2SO_4$ sur $H_2O$ total présent à la fin de la réaction est compris dans un intervalle allant de 1,3 à 4.

**13.** Procédé selon l'une quelconque des revendications 6 à 12, caractérisé en ce que la quantité de tétrahydrofuranne et/ou acétate d'éthyle utilisée n'est pas inférieure à 4 litres par mole de substrat.

**14.** Procédé selon l'une quelconque des revendications 6 à 13, caractérisé en ce que le solvant, tétrahydrofuranne et/ou d'acétate d'éthyle, est ajouté à une température qui n'est pas supérieure à 10 °C.

**15.** Utilisation de monopersulfates selon l'une quelconque des revendications 1 à 5 en tant qu'agents de blanchiment dans des compositions détergentes.

**Revendications pour l'Etat contractant suivant : ES**

**1.** Procédé de préparation de monopersulfates d'acides (poly)-percarboxyliques hétérocycliques azotés de formule générale (I):

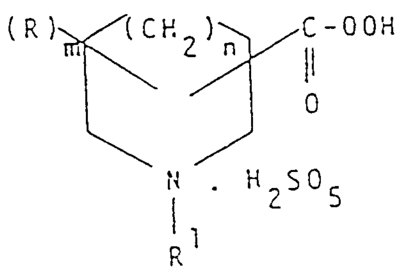

(I)

dans laquelle:

- R représente un atome d'hydrogène, ou un groupe alkyle linéaire ou ramifié, (hétéro)aryle, (hétéro)cycloalkyle, alkylaryle ou arylalkyle contenant jusqu'à 10 atomes de carbone, ces groupes étant éventuellement substitués par au moins un substituant choisi parmi F, Cl, $NO_2$ ou un groupe alcoxy en $C_1$-$C_5$; un groupe carboxylique ou un groupe percarboxylique; un atome de F, un atome de Cl et un groupe alcoxy en $C_1$-$C_5$, et dans laquelle les hétéroatomes sont sélectionnés parmi N et O;
- $R^1$ représente un groupe alkyle ayant plus de 5 atomes de carbone;
- n est un nombre entier choisi parmi 0, 1 et 2;
- m est un nombre entier choisi parmi 1, 2 et 3; et dans laquelle le cycle hétérocyclique peut lui-même être éventuellement condensé avec au moins un autre cycle (hétéro)aromatique ou (hétéro)cycloalkyle, les hétéroatomes étant sélectionnés parmi N et O,

caractérisé en ce que le substrat choisi parmi les acides (poly)carboxyliques hétérocycliques azotés et leur sel de N-sulfate, correspondant aux acides percarboxyliques désirés de formule (I), réagit avec $H_2O_2$ dans un milieu $H_2SO_4$ concentré et que le monopersulfate de formule générale (I) résultant est séparé du milieu réactionnel par addition de tétrahydrofuranne et/ou acétate d'éthyle.

**2.** Procédé selon la revendication 1, dans lesquels $R^1$ représente un groupe alkyle contenant de 8 à 20 atomes de carbone.

**3.** Procédé selon l'une quelconque des revendications 1 et 2, dans lesquelles n est égal à 1 et R est un atome d'hydrogène.

4. Procédé selon l'une quelconque des revendications 1 à 3 dans lesquelles le cycle hétérocyclique est condensé avec au moins un autre cycle (hétéro)aromatique ou (hétéro)cycloalkyle pyridinique.

5. Procédé selon la revendication 1, c'est-à-dire monopersulfate d'acide N-décyl-pipéridine-4-percarboxylique et monopersulfate d'acide N-hexadécyl-pipéridine-4-percarboxylique.

6. Procédé selon l'une quelconque des revendications 1 à 5, caractérisé en ce que l'acide (poly)-percarboxylique hétérocyclique azoté correspondant au composé désiré de formule (I) est converti en son sel d'$H_2SO_4$ correspondant qui réagit alors avec $H_2O_2$ dans $H_2SO_4$ concentré.

7. Procédé selon l'une quelconque des revendications 1 à 6, caractérisé en ce que l'acide (poly)-carboxylique hétérocyclique azoté ou son sel de N-sulfate réagit de façon graduelle avec $H_2O_2$ ayant une concentration de 70 à 90% en poids dans $H_2SO_4$ concentré à une température inférieure à 20°C.

8. Procédé selon l'une quelconque des revendications 1 à 7, caractérisé en ce que la quantité de $H_2SO_4$ est au moins égale à 5 moles par mole de substrat.

9. Procédé selon l'une quelconque des revendications 1 à 8, caractérisé en ce que la quantité de $H_2SO_4$ est comprise dans un intervalle allant de 6 à 15 moles par mole de substrat.

10. Procédé selon l'une quelconque des revendications 1 à 9, caractérisé en ce que la quantité de peroxyde d'hydrogène utilisée est au moins égale à 5 moles par mole de substrat.

11. Procédé selon l'une quelconque des revendications 1 à 10, caractérisé en ce que le rapport molaire final de $H_2SO_4$ sur $H_2O$ total présent à la fin de la réaction est compris dans un intervalle allant de 1,3 à 4.

12. Procédé selon l'une quelconque des revendications 1 à 11, caractérisé en ce que la quantité de tétrahydrofuranne et/ou acétate d'éthyle utilisée n'est pas inférieure à 4 litres par mole de substrat.

13. Procédé selon l'une quelconque des revendications 1 à 12, caractérisé en ce que le solvant, tétrahydrofuranne et/ou d'acétate d'éthyle, est ajouté à une température qui n'est pas supérieure à 10°C.

14. Utilisation de monopersulfates obtenus selon le procédé de l'une quelconque des revendications 1 à 5 en tant qu'agents de blanchiment dans des compositions détergentes.